# EUROPÄISCHE PATENTSCHRIFT

(11) **EP 3 257 478 B1**
(45) Veröffentlichungstag und Bekanntmachung des Hinweises auf die Patenterteilung: **09.01.2019**
(21) Anmeldenummer: 17178057.0
(22) Anmeldetag: 26.11.2013
(51) Int. Cl.: A61F 2/60, A61F 2/68, A61F 5/01, A61F 2/50, A61F 2/64, A61F 2/66, A61F 2/70, A61F 2/74, A61F 2/76

(54) **ORTHOPÄDIETECHNISCHE VORRICHTUNG**
ORTHOPAEDIC DEVICE
DISPOSITIF ORTHOPÉDIQUE

(30) Priorität: 26.11.2012 DE 102012023023
(43) Veröffentlichungstag der Anmeldung: 20.12.2017
(62) Teilanmeldung aus: 13817649.0
(73) Patentinhaber: Ottobock SE & Co. KGaA, 37115 Duderstadt (DE)
(72) Erfinder: Boiten, Herman, 37085 Göttingen (DE); Mosler, Lüder, 37115 Duderstadt (DE); Pusch, Martin, 37115 Duderstadt (DE); Te Riele, Frederik, 7557 JB Hengelo (NL); Michniewicz, Joachim, 80809 München (DE); Harbach, Lenka, 30173 Hannover (DE)
(74) Vertreter: Gramm, Lins & Partner Patent- und Rechtsanwälte PartGmbB

(56) Entgegenhaltungen:
- WO-A1-2012/177125
- FR-A1- 2 549 719
- GB-A- 245 577
- US-A1- 2009 265 018

## Beschreibung

Die Erfindung betrifft eine orthopädietechnische Vorrichtung zur orthetischen oder prothetischen Versorgung eines Patienten mit einem Kniegelenk, das ein proximales Oberteil und ein schwenkbar um eine Knieachse daran angeordnetes distales Unterteil aufweist, und einem Knöchelgelenk mit einer Knöchelgelenksachse, einem distal an dem Knöchelgelenk angeordneten, um die Knöchelgelenksachse verschwenkbaren Fußteil, einem zwischen dem Knöchelgelenk und dem Kniegelenk angeordneten Unterschenkelteil. Die Vorrichtung kann über eine proximal an dem Kniegelenk angeordnete Oberschenkelkomponente an dem Körper des Patienten festlegbar sein.

Orthesen haben die Aufgabe, die Bewegung einer vorhandenen Gliedmaße zu führen oder zu unterstützen bzw. eine Gliedmaße abzustützen und zu unterstützen, Prothesen ersetzen nichtvorhandene Gliedmaßen. Beinorthesen existieren in unterschiedlichen Ausführungsformen, sogenannte KAFO (knee angle foot orthosis) stützen sowohl den Fuß als auch das Knöchelgelenk und das Kniegelenk ab. Der Fuß wird in der Regel auf einer Fußplatte aufgesetzt, eine oder mehrere Unterschenkelschienen erstrecken sich parallel zum Unterschenkel, ein Kniegelenk ist ungefähr im Bereich der Knieachse vorgesehen, an eine oder mehrere Oberschenkelschienen sind Befestigungseinrichtungen zum Festlegen der Prothese an dem Oberschenkel angebracht. Ebenso können Befestigungseinrichtungen an den Unterschenkelschienen sowie der Fußplatte vorgesehen sein, um die Orthese an dem jeweiligen Bein festlegen zu können.

Protheseneinrichtungen mit einem Prothesenkniegelenk weisen einen Prothesenfuß auf, der über ein Unterschenkelrohr an dem Prothesenkniegelenk befestigt ist. Proximal zum Prothesenkniegelenk ist eine Befestigungseinrichtung für die Prothese vorgesehen, die üblicherweise als ein Oberschenkelschaft ausgebildet ist, in den der Oberschenkelstumpf eingeführt werden kann. Als Prothesenkniegelenk können unterschiedliche Bauformen verwendet werden, die von einem monoaxialen Prothesenkniegelenk über ein polyzentrisches Kniegelenk mit Dämpfungseinrichtungen zu computergesteuerten oder angetriebenen aktiven Prothesenkniegelenken reichen. Die Prothesenfüße können entweder starr oder gelenkig an dem Unterschenkelrohr befestigt sein, auch motorisch angetriebene Prothesenfüße sind möglich.

Eine orthopädietechnische Vorrichtung gemäß des Oberbegriffs von Anspruch 1 ist aus der GB 245 577 bekannt.

Die FR 2,549,719 A1 betrifft eine Prothese mit einem Prothesenkniegelenk und einem gelenkig an einem Unterschenkelrohr befestigten Prothesenfuß. Posterior zu der Knöchelgelenksachse ist eine Verbindungsstange angeordnet, die über einen Hebelmechanismus mit dem Prothesenkniegelenk gekoppelt ist. Wird das Prothesenkniegelenk eingebeugt, wird der Prothesenfuß angehoben und eine Dorsalflexion bewirkt.

Die US 2008/0269913 A1 betrifft ein Kunstbein mit einem Prothesenkniegelenk und einem Prothesenfuß. An dem Prothesenkniegelenk ist eine Verbindungsstange frontal zur Kniegelenksachse angelenkt, so dass bei einer Knieflexion die Verbindungsstange in einer Führung im Unterschenkelrohr verschoben wird. Über ein Zugelement wird die Bewegung zum Prothesenfuß geleitet, so dass die Fußspitze bei der Knieflexion angehoben wird.

Die US 2009/0265018 A1 betrifft eine angetriebene Beinprothese und ein Verfahren zur Steuerung der Beinprothese zum Erreichen eines nahezu natürlichen Gangbildes. Sowohl das Prothesenkniegelenk als auch das Knöchelgelenk sind mit separaten, motorischen Antrieben versehen. Über Sensoren wird in Echtzeit eine Steuerung auf der Grundlage verschiedener intramodaler Steuerungsprogramme durchgeführt.

Die EP 0 041 052 B1 betrifft eine Prothese für eine untere Gliedmaße, bei der ein Oberschenkelschaft und ein Unterschenkel über ein Verzahnungsgelenk miteinander gekoppelt sind. Eine federbelastete Kolbenstange hebt bei einer Knieflexion die Zehen an.

Die DE 47 53 03 B1 betrifft ein künstliches Bein, bei dem ein Unterschenkelteil mit einem Oberschenkelteil durch zwei Gelenkstäbe miteinander verbunden sind, um beim Anwinkeln des Prothesenkniegelenkes eine Dorsalflexion zu bewirken.

Bei Protheseneinrichtungen mit einer funktionellen Trennung von Kniegelenk und Knöchelgelenk wird während der Bewegung vielfach eine Dämpfung vorgesehen, so dass die kinetische Energie in Wärmeenergie umgewandelt wird. Existiert eine funktionelle Verbindung vom Kniegelenk zum Knöchelgelenk, wird die Bewegungsenergie vom Kniegelenk an das Knöchelgelenk geleitet.

Aufgabe der vorliegenden Erfindung ist es, bei möglichst geringem konstruktivem Aufwand eine Kopplung zwischen dem Kniegelenk und dem Knöchelgelenk herbeizuführen, mit der eine Nutzung der Bewegungsenergie des Knies für die Knöchelbewegung möglich ist, die eine Annäherung an das natürliche Gangbild bewirkt und am Ende der Standphase eine Vertikalbewegung des Körperschwerpunktes minimiert.

Erfindungsgemäß wird dies durch eine orthopädietechnische Vorrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die erfindungsgemäße orthopädietechnische Vorrichtung zur orthetischen oder prothetischen Versorgung eines Patienten mit einem Kniegelenk, das ein proximales Oberteil und ein schwenkbar, insbesondere um eine feste oder momentane Knieachse verschwenkbar, daran angeordnetes distales Unterteil aufweist, und einem Knöchelgelenk, das insbesondere eine feste oder momentane Knöchelgelenksachse aufweist, einem distal an dem Knöchelgelenk angeordneten, verschwenkbaren Fußteil und einem zwischen dem Knöchelgelenk und dem Kniegelenk angeordneten Unterschenkelteil sieht vor, dass das Oberteil des Kniegelenkes oder das daran befestigte Oberschenkelteil mit einem Fußteil über eine Kraftübertragungseinrichtung gekoppelt ist, die bei einer Knieflexion eine Plantarflexion des Fußteils bewirkt. Durch die Kopplung des Oberteils des Kniegelenkes oder einer proximal an dem Kniegelenk angeordneten Oberschenkelkomponente, die an dem Körper des Patienten festlegbar ist, beispielsweise eine Oberschenkelschiene oder ein Oberschenkelschaft, mit einem Fußteil über eine Kraftübertragungseinrichtung, die bei einer Knieflexion eine Plantarflexion des Fußteils bewirkt, ist es möglich, die Bewegungsenergie des Knies für die Knöchelbewegung zu nutzen. Analog zu dem natürlichen Gangbild, bei dem am Ende der Standphase eine Plantarflexion vorgenommen wird, um die Beinlänge bei der Kniebeugung zu verlängern, wird durch die Kraftübertragungseinrichtung das Fußteil plantar flektiert. Dadurch wird die Dauer des Bodenkontaktes des Fußteils verlängert, wodurch die Vertikalbewegung des Körperschwerpunktes minimiert wird. Durch die energetische Kopplung der Knieflexion mit der Plantarflexion des Fußteils wird eine hohe energetische Effizienz bei einem geringen apparativen Aufwand erreicht. Wird während der Schwungphase das Kniegelenk extendiert, kann eine Bewegungsumkehr erfolgen, so dass eine Dorsalflexion des Fußteils veranlasst wird. Ebenso kann nach Erreichen eines vorbestimmten Beugewinkels eine Umschaltung der Aktuierung des Prothesenfußes erfolgen, so dass nach Erreichen des Beugewinkels eine Dorsalflexion durchgeführt wird, um die effektive Beinlänge zu verringern und in der Schwungphase, insbesondere beim Vorbringen des Fußes, ein Stolpern oder Hängenbleiben an Hindernissen zu vermeiden. Unter einer Plantarflexion wird dabei diejenige Bewegung verstanden, bei der eine Verschwenkung des Fußteils in Richtung auf den Boden erfolgt, so dass sich der Winkel zwischen dem Unterschenkelteil und dem Fußteil vergrößert. Die Dorsalflexion ist die entgegengesetzte Bewegung, bei der der Fußrücken oder Spann in Richtung auf das Schienbein oder das Unterschenkelteil bewegt wird und sich der Winkel zwischen dem Fußrücken oder Spann und dem Unterschenkelteil verringert. Die verschenkbare Verbindung zwischen den einzelnen Komponenten, kann über eine einzige feste Achse realisiert werden, also eine Achse, die nur eine Drehbewegung erlaubt und die an zumindest einer der Komponenten lagestabil befestigt ist. Alternativ kann bei polyzentrischen Gelenken eine verlagerbare Momentandrehachse realisiert sein, die aufgrund der polyzentrischen Lagerung bei Mehrlenkergelenken nicht relativ zu zumindest einer Komponente ortsfest ist, sondern im Verlauf der Verschwenkbewegung wandert. Es entsteht so eine virtuelle Drehachse, um die das Oberteil gegenüber dem Unterteil oder das Unterschenkelteil gegenüber dem Fußteil verschwenkt. Es ist auch möglich, dass die verschwenkbare Anbindung zweier Komponenten aneinander über ein Elastomergelenk erfolgt, so dass keine fest definierte Drehachse vorhanden ist, sondern sich innerhalb des Elastomergelenks bei unterschiedlichen Belastungen unterschiedliche Drehachse ausbilden, beispielsweise bei unterschiedlichen Querkräften oder bei Torsionskräften um eine Achse, die in proximal-distal-Richtung verläuft.

Beispielsweise kann die Kraftübertragungseinrichtung als hydraulisches System ausgebildet sein, so dass über Kolbenstangen, hydraulische Leitungen und Ventile eine sehr platzsparende, leichte Kraftübertragung von dem Kniegelenk zum Fußteil erfolgen kann. Ebenso kann die Umschaltung von Plantarflexion auf Dorsalflexion sehr leicht über ein Umschaltventil durchgeführt werden, das mechanisch oder auch elektrisch angesteuert werden kann.

Erfindungsgemäß ist eine mechanische Koppeleinrichtung vorgesehen, die zugkraft- und/oder druckkraftübertragend ausgebildet sein kann. Mechanische Koppeleinrichtungen haben den Vorteil einer robusten Bauweise mit einer hohen Verfügbarkeit und einfachen Reparaturmöglichkeit. Aufgrund der bewegten Teile bei einer mechanischen Kopplung können Kleidungsstücke oder prothetische Verkleidungen scheuern oder eingeklemmt werden.

Die mechanische Koppeleinrichtung kann an einer dorsal oder ventral zu der Knieachse hinausstehenden ersten Lagerungsstelle gelagert und an einer ventral oder dorsal zu der Knöchelgelenksachse zweiten Lagerstelle gelagert sein, wobei die Lagerungsstellen auf unterschiedlichen Seiten der einen Verbindungslinie zwischen einer Knieachse und einer Knöchelachse liegen. Bei einer dorsal überstehenden ersten Lagerungsstelle wird die Koppeleinrichtung bei einer Knieflexion auf Druck belastet und überträgt die Kräfte bei der Knieflexion am Ende der Standphase auf die ventral zu der Knöchelgelenksachse liegenden zweiten Lagerungsstelle, so dass ein Moment um die Knöchelgelenksachse entsteht. Bei einer umgekehrten Anordnung der ersten Lagerungsstelle, also ventral zu der Knieachse, wird die Momentenerzeugung über eine zugkraftübertragende Koppeleinrichtung realisiert.

Die mechanische Koppeleinrichtung ist als Gelenkstab ausgebildet. Bei einer Ausgestaltung als Gelenkstab ist es möglich, dass bis zu einem gewissen Kniewinkel der Gelenkstab eine Druckkraftübertragung oder auch Zugkraftübertragung zulässt, ab dem Erreichen des vorbestimmten Kniewinkels, wird der Gelenkstab in dem Gelenk geknickt, so dass keine substanzielle Kraftübertragung mehr erfolgen kann oder eine Kraftrichtungsumkehr erfolgt. Der Kniegelenkswinkel, bei dem der Gelenkstab einknickt, bestimmt sich aus den geometrischen Beziehungen zwischen dem oberen Anlenkpunkt der Koppeleinrichtung und dem unteren Anlenkpunkt sowie gegebenenfalls einer Vorspannung des Gelenkes. Der Gelenkstab ist nur in einer Richtung, ausgehend von einer Ausgangsstellung, einknickbar, so dass vor dem Erreichen des vorbestimmten Kniegelenkwinkels eine sichere Kraftübertragung stets möglich ist und eine Rückkehr in die Ausgangsposition sicher erfolgen kann. Das Knöchelgelenk kann unter einer Vorspannung in Dorsalflexionsrichtung stehen, um bei einem Wegfall der Krafteinleitung über die Koppeleinrichtung oder das Hydrauliksystem ohne Kraftrichtungsumkehr eine Dorsalflexion bewirken zu können.

Um einer Einstellung an die Wünsche des Patienten und dessen körperliche Gegebenheiten vornehmen zu können, ist zumindest die erste Lagerungsstelle einstellbar ausgebildet, so dass der Abstand der ersten Lagerungsstelle von der Kniegelenksachse oder der Knöchelgelenksachse einstellbar ist. Neben dem Abstand kann auch die Position zu der jeweiligen Achse einstellbar ausgestaltet sein, um den Grad der Verlagerung bzw. Plantarflexion des Fußteils in Abhängigkeit von der Knieflexion einstellen zu können. Je nach Hebelverhältnis kann eine größere oder geringere Plantarflexion eingestellt werden.

Bei einer nichterfindungsgemäßen hydraulischen Kraftübertragungseinrichtung können eine Steuerungseinrichtung und ggf. zumindest ein Schaltventil vorgesehen sein, um eine Kraftübertragung zu ermöglichen oder zu unterbrechen. Die Unterbrechung oder das Ermöglichen der Kraftübertragung kann auf der Grundlage von Sensorwerten, einer Winkelstellung und/oder einer besonderen Belastungssituation erfolgen, beispielsweise in Abhängigkeit von dem Kniewinkel, dem Knöchelgelenkswinkel oder gemessenen Kräften oder Momenten. Die Steuerungseinrichtung kann entweder mechanisch oder elektrisch gesteuert sein. Bei einer mechanischen Steuerung kann eine Steuereinrichtung beispielsweise an dem Kniegelenk angeschlossen und beispielsweise als eine Steuerscheibe, ein Steuernocken oder dergleichen ausgebildet sein, so dass in Abhängigkeit von dem erreichten Kniewinkel eine bestimmte hydraulische Reaktion eingeleitet wird. Eine korrespondierende Ausgestaltung kann für das Knöchelgelenk vorgesehen sein. Ab Erreichen eines bestimmten Winkels kann dann die Kraftübertragung unterbrochen werden oder eine Bewegungsumkehr per Umschaltung dergestalt erfolgen, dass eine Dorsalflexion bei einer Erreichen eines vorbestimmten Kniewinkels eingeleitet wird. Auch bei einer Bewegungsumkehr des Unterschenkelteils in der Schwungphase kann Ventilstellung durch Umschaltung dergestalt erfolgen, dass eine Dorsalflexion bei einer Extensionsbewegung des Kniegelenks bewirkt wird. Werden Sensoren eingesetzt, die bestimmte Belastungen, Bewegungen, Winkel oder andere Messgrößen erfassen, können die erfassten Sensorwerte genutzt werden, um die Kraftübertragung zu steuern, also eine Kraftübertragung zu ermöglichen, zu unterbrechen oder nur einen Teil der Kräfte zu übertragen. Die Sensoren sind mit der Steuereinrichtung gekoppelt, die die Sensorsignale aufnimmt, verarbeitet und über einen Aktuator beispielsweise ein Ventil verstellt, also öffnet oder schließt, um einen Fluidstrom zu verändern.

An dem Oberteil oder an dem Oberschenkelteil sowie an dem Unterschenkelteil oder dem Unterteil oder Fußteil kann je eine Zylinderkolbeneinheit angeordnet sein, die durch zumindest eine Hydraulikleitung miteinander verbunden sind. Durch die Kniebewegung wird Druck auf die obere Hydraulikeinheit ausgeübt, der über eine entsprechend Leitung und gegebenenfalls einen Ventilblock zur unteren Hydraulikeinheit geleitet wird, um das Fußteil zu aktuieren.

Die Zylinderkolben-Einheiten können über Ventile parallel oder überkreuzt miteinander verbunden sein, ebenso ist es möglich, dass eine individuelle Einstellung bis zur Sperrung der Zylinder-Kolbeneinheiten durch die Steuerungseinrichtung vorgenommen wird, um in Abhängigkeit der jeweiligen Kniestellung, Bewegungsrichtung, Bewegungsgeschwindigkeit, Bewegungsbeschleunigung oder Gangsituation eine Freigabe oder eine Aktuierung des Fußteils zu ermöglichen.

Es kann eine Sensoreinrichtung zur Erfassung des Kniewinkels, der Winkelgeschwindigkeit oder Winkelbeschleunigung vorgesehen sein, die mit einer Steuereinrichtung zum Verstellen eines Ventils oder mehrere Ventile verbunden ist, so dass eine elektronisch gesteuerte Ventilschaltung vorgesehen ist, um die hydraulische oder mechanische Kraftübertragung zu steuern.

Die Kraftübertragungseinrichtung kann eine Kraftübertragung in Dorsalflexionsrichtung sperren, um zu verhindern, dass eine Verlagerung während der Standphase zu einer ungewollten Knieeinbeugung führt. Es kann vorgesehen sein, dass die maximale Dorsalflexionsstellung variabel einstellbar ausgebildet ist, also eine Dorsalflexionsanschlag vorgesehen ist, der verstellbar ist, um eine Anpassung an den jeweiligen Nutzer oder die Nutzungsbedingen vornehmen zu können. Der Dorsalflexionsanschlag kann als mechanischer Anschlag ausgebildet sein oder durch Schließen eines Ventils verwirklicht werden.

Eine Rückstellvorrichtung kann dem Fußteil zugeordnet sein, um eine Dorsalflexion des Fußteils zu bewirken. Die Rückstellvorrichtung kann beispielsweise als eine Feder oder Elastomerelement ausgebildet sein, die einer Plantarflexion entgegenwirkt. Das Knöchelgelenk kann als elastisches Gelenk ausgebildet sein, in dem ein elastisches Element, beispielsweise ein Elastomerkörper, angeordnet ist, wobei durch das elastische Gelenk eine Rückstellkraft auf das Fußteil ausgeübt wird, so dass das Fußteil ohne Einwirkung äußerer Kräfte in eine Ausgangsstellung zurückbewegt wird.

Eine Ausführungsform sieht vor, dass in dem Knöchelgelenk zumindest ein Dämpfer vorhanden ist, der einer Dorsalflexion entgegenwirken, so dass eine Rückstellbewegung nach einer Plantarflexion ohne Überschwingen ausgeführt werden kann.

Nachfolgend werden Ausführungsbeispiele anhand der beigefügten Figuren näher erläutert. Es zeigen:
Es zeigen:
Fig. 1 Knöchel- und Kniewinkelverläufe;
Fig. 2 eine perspektivische Ansicht einer Vorrichtung;
Fig. 3 eine Seitenansicht der Vorrichtung gemäß Figur 2 in gestreckter Stellung;
Fig. 3a eine perspektivische Darstellung gemäß Fig. 3;
Fig. 4 eine Vorrichtung gemäß Figur 3 in eingebeugter Stellung;
Fig. 5 eine Prinzipdarstellung einer hydraulischen Kraftübertragung;
Fig. 6 eine schematische Darstellung einer Protheseneinrichtung mit einer Hydraulikeinheit;
Fig. 7 eine perspektivische Darstellung der Ausführungsform gemäß Figur 6;
Fig. 8 eine gespiegelte Darstellung der Ausführungsform gemäß Figur 6;
Fig. 8a eine Schnittansicht der Fig. 8;
Fig. 9 eine Schnittdarstellung einer Zylinder-Kolben-Einheit;
Fig. 10 eine perspektivische Gesamtansicht einer Zylinder-Kolben-Einheit;
Fig. 11 eine Querschnittsdarstellung einer eingebauten Zylinder-Kolben-Einheit im gestreckten Zustand;
Fig. 12 eine Vorrichtung gemäß Figur 11 mit einem Beugewinkel von 60°;
Fig. 13 eine Vorrichtung gemäß Figur 11 mit einem Beugewinkel von 120°;
Fig. 14 eine Schnittdarstellung einer flächenseitigen Zylinder-Kolben-Einheit in dorsal flektierter Stellung;
Fig. 15 eine Ausführungsform gemäß Figur 14 in plantar flektierter Stellung;
Fig. 16 eine schematische Darstellung unterschiedlicher Schaltmöglichkeiten;
Fig. 17 eine schematische Darstellung einer Ventilanordnung;
Fig. 18 Kniewinkel- und Knöchelgelenkswinkeldarstellungen über der Zeit; sowie
Fig. 19 überlagerte Knöchelgelenkswinkelverläufe über der Zeit.

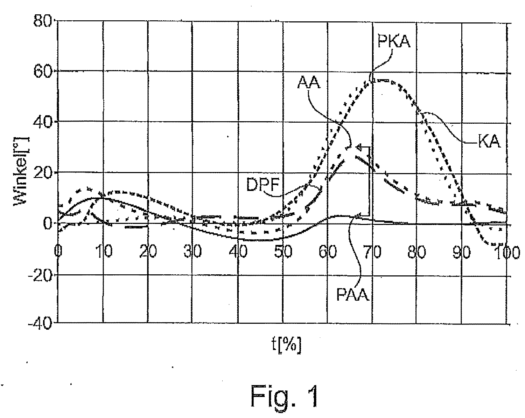

In der Figur 1 ist eine Darstellung unterschiedlicher Winkel über die Zeit während eines Gangzyklusses dargestellt. Der natürliche Kniewinkel KA und der natürliche Knöchelwinkel AA ist in gestrichelten Linien dargstellt, der Prothesenkniewinkel PKA und der Prothesenknöchelwinkel PAA sind in den durchgezogenen Linien über die Zeit eines Schrittes vom Aufsitzen der Ferse bis zum erneuten Aufsetzen dargestellt. Während der natürliche Kniewinkel KA sehr gut durch eine Protheseneinrichtung angenähert realisiert werden kann, was sich aus dem geringen Differenzwinkel und einem nahezu deckungsgleichen Verlauf der Kniewinkel KA und der Prothesenkniewinkel PKA ergibt, ist der Prothesenknöchelwinkel PAA stark abweichend gegenüber dem natürlichen Knöchelwinkel AA, das Maß der Abweichung wird durch den Differenzwinkel ΔPF angezeigt. Die Maximalabweichung besteht bei ungefähr zwei Drittel des Gangzyklusses kurz vor der Zehenablösung, dem sogenannten toe off, bei dem bei einer natürlichen Gangphase die maximale Plantarflexion bei ca. 30° ausgehend von der Ausgangsstellung im Stehen liegt, wohingegen die maximale Plantarflexion bei einer Prothese bei ungefähr 2° liegt, was durch die Dynamik des Fußes bedingt ist.

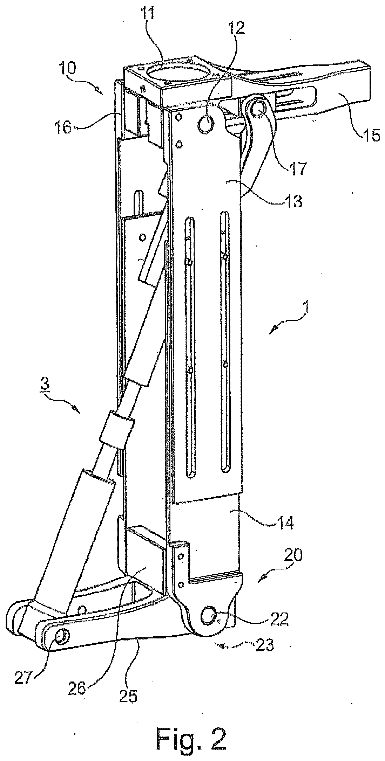

Um ein möglichst natürliches Gangbild bereitzustellen, wird bei einer ersten Ausführungsform eine orthopädietechnische Vorrichtung 1 in Gestalt einer Unterschenkelprothese gemäß Figur 2 vorgeschlagen, die ein Kniegelenk 10 mit einem proximalen Oberteil 11 und einem gelenkig um eine Knieachse 12 daran befestigten Unterteil 13 aufweist. An dem distalen Unterteil 13 ist ein Unterschenkelteil 14 befestigt, das eine Verbindung zu dem Knöchelgelenk 20 herstellt und um eine Knöchelgelenksachse 22 schwenkbar an einem Fußteil 23 gelagert ist. Das Fußteil 23 ist im dargestellten Ausführungsbeispiel als Aufnahme für einen Justierkern für einen Prothesenfuß ausgebildet und weist einen Ausleger 25 auf, der im dargestellten Ausführungsbeispiel in Gehrichtung nach vor gerichtet, also anterior orientiert ist. Das Unterteil 13 und das Unterschenkelteil 14 weisen Schlitzführungen auf, so dass die beiden Komponenten verschieblich aneinander gelagert sind, um eine Längenanpassung vornehmen zu können, falls dies gewünscht oder erforderlich ist. Das Unterteil 13 besteht aus zwei Seitenplatten, die an ebenfalls als Seitenplatten ausgebildete Unterschenkelteile 14 befestigt sind, die Seitenplatten liegen deckend aufeinander. An dem Unterteil 13 ist ein Anschlag 16 zur Begrenzung des maximalen Extensionswinkels vorgesehen, ebenso ist am Unterschenkelteil 14 ein Knöchelanschlag 26 vorgesehen, der die maximale Dorsalflexion des Fußteils 23 begrenzt.

An dem Oberteil 11, das um die Knieachse 12 in Uhrzeigerrichtung verschwenkbar gelagert ist, ist ein nach in Gehrichtung nach hinten weisender, also posterior orientierter Ausleger 15 angeordnet, in dem eine Langlochführung ausgebildet ist, in der ein distaler Anlenkpunkt 17 für eine Kraftübertragungseinrichtung 3 gelagert ist. Der Anlenkpunkt 17 ist verschieblich innerhalb der Langlochführung angeordnet und wird dort von einem Orthopädietechniker in der für den Patienten korrekten Position fixiert. Der konkrete Aufbau der Kraftübertragungseinrichtung 3, die im dargestellten Ausführungsbeispiel als mechanisches Koppelelement in Gestalt einer Gelenkstange ausgebildet ist, wird später näher erläutert werden.

Das untere Ende des Koppelelementes ist in einem distalen Anlenkpunkt 27 an dem distalen Ausleger 25 gelenkig, jedoch unverschieblich gelagert. Durch die Anordnung der Anlenkpunkte 17, 27 posterior und anterior einer Verbindungslinie zwischen der Knieachse 12 und der Knöchelgelenksachse 22 führt eine Einbeugung des Kniegelenks 10 aufgrund der Rotation des exzentrisch zur Knieachse 12 gelagerten proximalen Anlenkpunktes 17 in Uhrzeigerrichtung zu einer Verschiebung des Koppelelementes und einer Drehung des distalen Auslegers 25 um die Knöchelgelenksachse 22 entgegen der Uhrzeigerrichtung, so dass das Fußteil 23 mit dem daran befestigten, nicht dargestellten Prothesenfuß bei einer Knieflexion in Plantarrichtung flektiert wird.

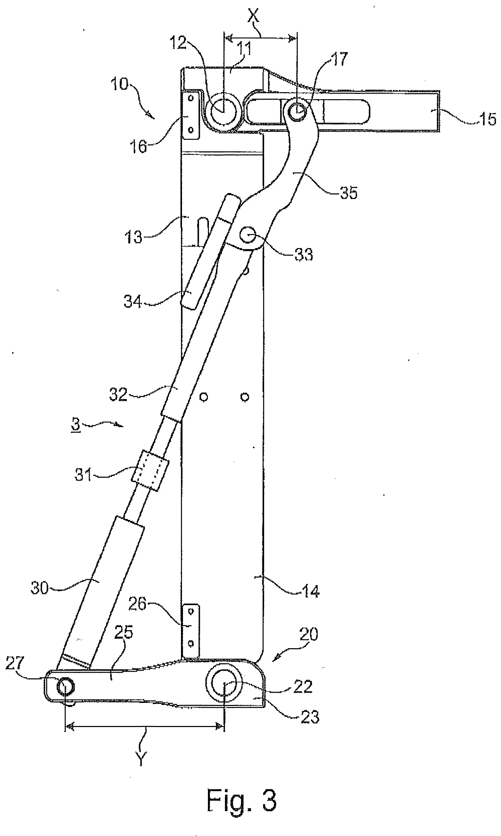

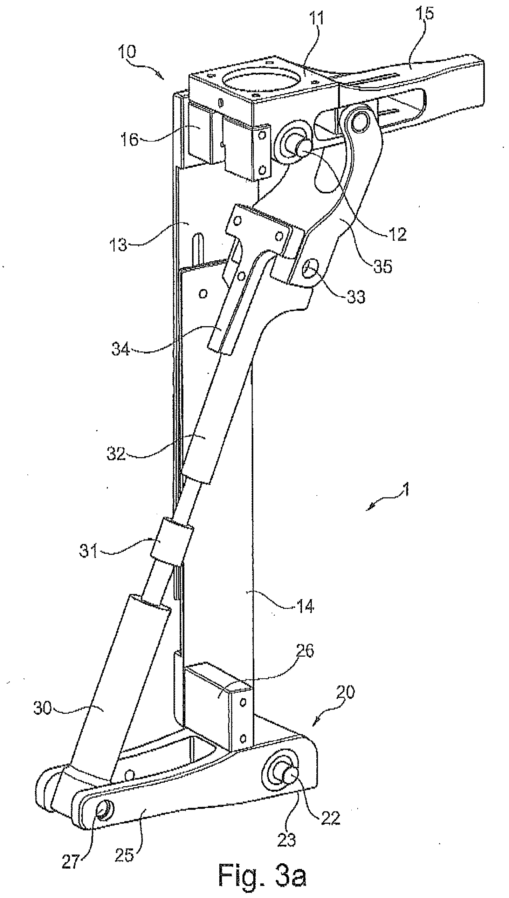

In der Figur 3 ist in einer seitlichen Schnittdarstellung die Prothese 1 gemäß Figur 2 in einer Ausgangsstellung gezeigt, die dem Stehen entspricht. Das Kniegelenk 10 befindet sich in der maximalen Extension und der Ausleger 25 des Knöchelgelenks 20 steht im Wesentlichen rechtwinklig zu der Längserstreckung sowohl des Unterschenkelteils 14 als auch des Unterteils 13 und der Verbindungslinie zwischen der Knieachse 12 und der Knöchelgelenksachse 22.
An dem distalen Ausleger 15 ist die Langlochführung für den proximalen Anlenkpunkt 17 zu erkennen, ebenso wie die proximale Hebellänge X, die variabel eingestellt werden kann. An dem distalen Ausleger 25 ist die Knöchelhebellänge Y, die die den Abstand zwischen dem distalen Anlenkpunkt 27 und der Knöchelgelenksachse 22 darstellt, eingezeichnet. Gut zu erkennen ist ebenfalls die mechanische Kraftübertragungseinrichtung 3 mit einer distalen Hülse 30, die gelenkig an dem Knöchelausleger 25 gelagert ist, einer Doppelschraube 31 mit gegenläufigen Gewinden sowie einer proximalen Hülse 32, die um eine Gelenkachse 33 schwenkbar an einer Spange 35 gelagert ist. An der Spange 35 ist ein Anschlag 34 ausgebildet, der verhindert, dass die proximale Hülse 32 in Uhrzeigerrichtung um die Achse 33 über einen vorbestimmten Winkel hinaus auslenkt.

In der Figur 3a ist in einer perspektivischen Darstellung gemäß Figur 3 die Prothese 1 gezeigt. In der perspektivischen Darstellung ist gut zu erkennen, dass der Knöchelausleger 25 als eine einseitig offene Gabel ausgebildet ist, die zwei Schenkel aufweist, zwischen denen die distale Hülse 30 an dem distalen Anlenkpunkt 27 verschwenkbar gelagert ist. Sowie die distale Hülse 30 als auch die proximale Hülse 32 weisen einen Innengewinde auf, in das die Doppelschraube 31 eingeschraubt ist. Die proximale Hülse 32 ist um die Gelenkachse 33 an der Spange 35 gelagert, die wiederum an dem proximalen Ausleger 15 befestigt ist. Das Oberteil 10 ist über Zapfen gelenkig um die Schwenkachse 12 an dem Unterteil 13 gelagert, der Anschlag 34 verhindert ein Verschwenken der proximalen Hülse 32 in Uhrzeigerrichtung um die Gelenkachse 33 über das festgelegte Maß hinaus, das durch den Anschlag 34 bestimmt ist.

An dem Unterschenkelteil 14 ist der mechanische Dorsalflexionsanschlag 26 befestigt, über den die maximale Dorsalflexion des Fußteils 23 festgelegt wird. Der Dorsalflexionsanschlag 26 ist verstellbar ausgebildet, so dass die maximale Dorsalflexion variabel einstellbar ist.

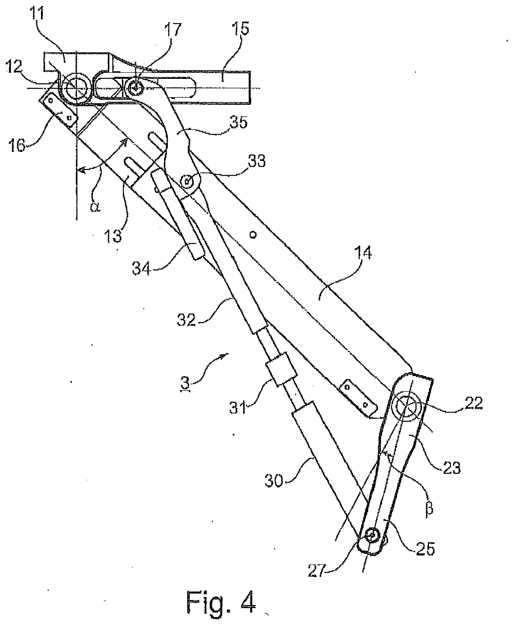

In der Figur 4 ist die Prothese 1 in einem eingebeugten Zustand gezeigt. Während in der Figur 3 der Kniewinkel α 0° betrug, ist in der Figur 4 ein Kniewinkel von ungefähr 50° vorgesehen. Der Plantarflexionswinkel β, der in der Stellung gemäß der Figur 30° betrug, beträgt in der Figur 4 ca. 25°, was über die verschiedenen Hebellängen X, Y in den jeweiligen Auslegern 15, 25 variiert werden kann. Wenn auch die Hebellänge Y am Knöchelausleger 25 einstellbar ist, kann das Übersetzungsverhältnis im Zusammenspiel mit einer Längenanpassung über die Doppelschraube 31 einfach und vielfältig durchgeführt werden. Die Ausgangsstellung, das heißt der Plantarflexionswinkel β in Abhängigkeit von dem Kniewinkel α in der Stellung gemäß Figur 3, kann über die Doppelschraube 31 sowie die Position des proximalen Anlenkpunktes 17 innerhalb der Langlochführung eingestellt werden. Wird die Doppelschraube 31 aus den Gewinden in den Hülsen 30, 32 herausgedreht, verlängert sich die Kraftübertragungseinrichtung 3, so dass bei einer gleichbleibenden Position der Anlenkpunkte 17, 27 das Fußteil 23 in Plantarrichtung flektiert wird, umgekehrt wird bei entgegengesetzter Drehrichtung eine Dorsalflexion eingeleitet.

In der Figur 4 ist der für das Ausführungsbeispiel maximale Kniewinkel α dargestellt, bis zu der über die Kraftübertragungseinrichtung aus den beiden Hülsen 30, 32, der Doppelschraube 31 sowie der Spange 35 eine Druckkraft auf den distalen Ausleger 25 übertragen werden kann, ohne dass die distale Hülse 32 um die Achse 33 verschwenkt. In der Spange 35 kann ein Anschlag ausgebildet sein, ebenfalls kann eine weitere Rotation der Spange 35 relativ zu dem Ausleger 15 durch andere Einrichtungen verhindert werden, so dass bei einer weiteren Einbeugung im Kniegelenk 10 das distale Ende der Spange 35 in Uhrzeigerrichtung verlagert wird, so dass ein Einknicken in der Kraftübertragungseinrichtung 3 stattfindet, was zu einer Verkürzung der effektiven Länge zwischen den beiden Anlenkpunkten 17, 27 führt. Dies führt zu einer Verringerung des Plantarflexionswinkels β, was durch eine Vorspanneinrichtung, beispielsweise in Gestalt einer Feder, unterstützt werden kann. Durch die Verringerung des Plantarflexionswinkels β wird die effektive Beinlänge verringert, indem die Fußspitze des nicht dargestellten Prothesenfußes in Dorsalflexionsrichtung bewegt und angehoben wird, so dass ein einfacheres Durchschwingen in der Schwungphase erfolgen kann, wenn eine Extensionsbewegung im Kniegelenk 10 durchgeführt wird.

Grundsätzlich ist es auch möglich, die Ausleger 15, 25 in die jeweils umgekehrte Richtung zu orientieren, also den proximalen Ausleger 15 in Anteriorrichtung und den distalen Ausleger 25 in Posteriorrichtung, die Kraftübertragungseinrichtung 3 arbeitet dabei im Wesentlichen zugkraftübertragend, wenn das Kniegelenk 10 eingebeugt wird.

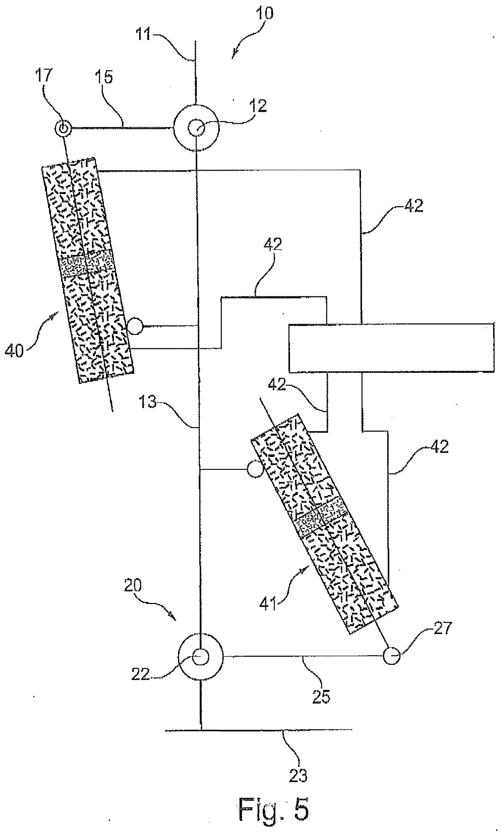

In der Figur 5 ist ein prinzipieller Aufbau einer hydraulischen Kraftübertragungseinrichtung 3 gezeigt. Auch hier sind Ausleger 15, 25 an dem Kniegelenk 10 und dem Knöchelgelenk 20 vorgesehen, bei einer sehr einfachen Ausführung des Kniegelenks 10 kann das Unterteil 13 gleichzeitig die Verbindung zwischen dem Kniegelenk 10 und dem Knöchelgelenk 20 ausbilden. In der Regel ist das Kniegelenk 10 als separates Bauteil ausgebildet und weist ein Oberteil 11 und ein Unterteil 13 auf, an dem über Befestigungsmittel ein Unterschenkelrohr oder Unterschenkelteil 14 befestigbar ist, das wiederum an Befestigungsmitteln des Knöchelgelenks 20 eines Prothesenfußes festgelegt ist. Im dargestellten Ausführungsbeispiel ist eine erste hydraulische Zylinder-Kolbeneinheit 40 an dem proximalen Anlenkpunkt 17 sowie an dem Unterteil 13 gelagert. Über Hydraulikleitungen 42, die in einen Ventilblock 43 münden, steht eine distale Zylinder-Kolbeneinheit 41 mit der proximalen Zylinder-Kolbeneinheit 40 in strömungstechnischer Verbindung. Die distale Kolben-Zylindereinheit 41 ist gegebenenfalls an dem Unterteil 13 oder einem Unterschenkelteil sowie an dem Ausleger 25 an dem distalen Anlenkpunkt 27 gelagert. Wird das Kniegelenk 10 eingebeugt, verfährt der Kolben innerhalb der proximalen Kolben-Zylindereinrichtung 40 nach unten, Hydraulikfluid wird durch die Leitung 42 in den Ventilblock 43 und von dort in die obere Kammer der distalen Kolben-Zylindereinheit 41 geleitet, so dass eine Plantarflexion des Fußteils 23 um die Knöchelgelenksachse 22 bewirkt wird.

Im dargestellten Ausführungsbeispiel sind die beiden Hydraulikeinheiten 40, 41 als zwei Gleichlaufzylinder ausgebildet, die von den Volumina und Durchmessern gleich ausgestaltet sind. Eine individuelle Übersetzung kann durch unterschiedliche Hebel an dem Kniegelenk 10 und dem Knöchelgelenk 20 realisiert werden.

In dem Ventilblock 43 können mehrere und unterschiedliche Ventile vorgesehen sein, um alle Zylindervolumina miteinander beliebig zu verschalten, um bei gewünschten Kniewinkelstellungen verschiedene Plantarflexionswinkel β einstellen zu können. Innerhalb des Ventilblocks 43 können Proportionalventile vorhanden sein, über die es möglich ist, Drosselungen vorzunehmen, so dass unterschiedliche hydraulische Widerstände bereitgestellt werden können. Durch unterschiedliche Verschaltungen der einzelnen Zylinder kann je nach Bewegungsart und Bewegungsphase eine gewünschte Verstellung des Fußteils 23 in Abhängigkeit von dem Kniewinkel α realisiert werden.

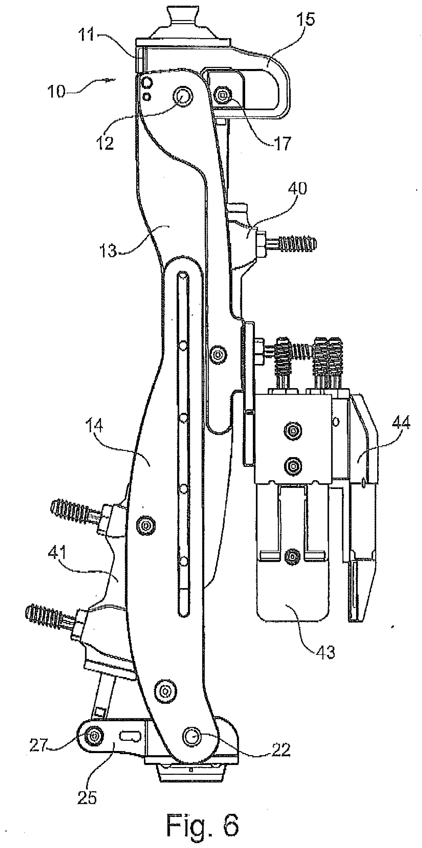

In der Figur 6 ist eine schematische Seitenansicht einer Protheseneinrichtung mit hydraulischer Kraftübertragung dargestellt. Ähnlich wie bei dem Ausführungsbeispiel gemäß Figur 2 ist im proximalen Ausleger 15 eine Langlochführung vorgesehen, in der der obere Anlenkpunkt 17 der proximalen Kolben-Zylindereinheit 40 gelagert ist. Über eine Fixiereinrichtung kann die Hebellänge X und damit die mechanische Übersetzung eingestellt werden. An dem Oberteil 11 des Kniegelenks 10 ist ein Pyramidenadapter befestigt, an dem Unterteil 13 des Kniegelenks 10 mit den Seitenplatten sind höhenverstellbare Unterschenkelteile 14 gelagert, zwischen denen die distale Kolben-Zylindereinheit 41 angeordnet ist. Der untere Anlenkpunkt 27 ist drehbar aber jedoch unverstellbar an dem distalen Ausleger 25 gelagert, an dem Sensoren, beispielsweise in Gestalt von Dehnmessstreifen angeordnet sein können, um die Belastung zu erfassen. Der Ventilblock 43 ist mit einer Steuereinrichtung 44 versehen, in der eine Schaltelektronik untergebracht ist, so dass die innerhalb des Ventilblocks 43 angeordneten Ventile zur unterschiedlichen Kopplung der Zylinder in den Zylinder-Kolbeneinheiten 40, 41 individuell und nach Auswertung von Sensordaten angesteuert werden können.

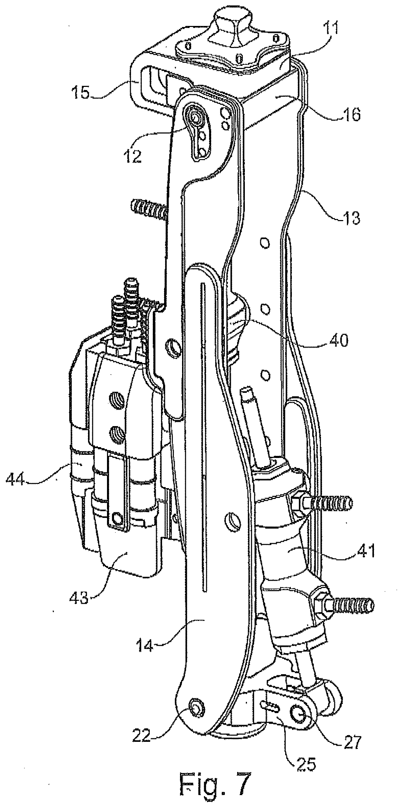

In der Figur 7 ist eine perspektivische Darstellung des Ausführungsbeispiels gemäß Figur 6 gezeigt, aus der zu entnehmen ist, dass die Hydraulik-Zylindereinheiten 40, 41 zwischen den als Seitenplatten ausgebildeten Unterschenkelteilen 14 und Unterteilen 13 angeordnet ist. Ebenfalls kann der Ventilblock 43 zwischen den Seitenplatten angeordnet sein.

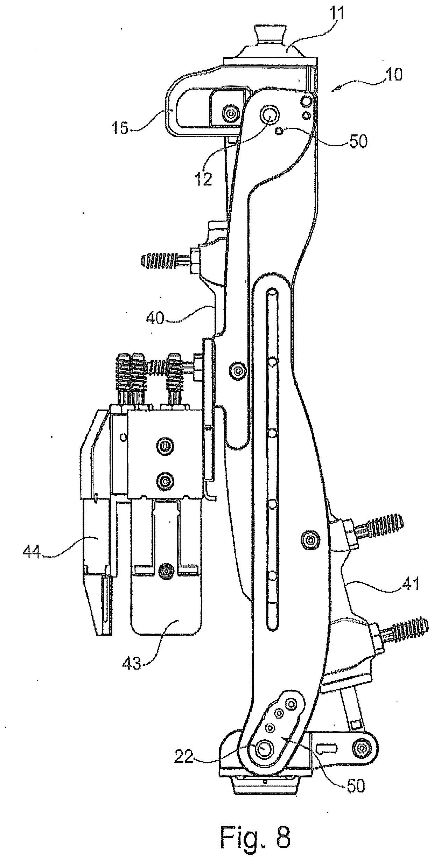

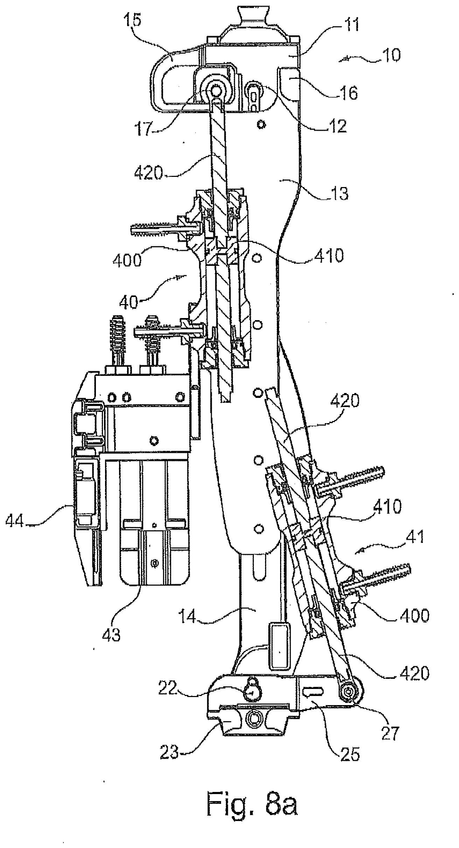

In der Figur 8 ist eine Variante gezeigt, bei der sowohl im Kniegelenk 10 als auch im Knöchelgelenk 20 Winkelsensoren 50 mit der Steuereinheit 44 gekoppelt sind, so dass in Abhängigkeit von dem Kniewinkel α und dem Plantarflexionswinkel β eine Verschaltung der Ventile innerhalb des Ventilblocks 43 winkelabhängig durchgeführt werden kann. Ebenfalls ist es möglich, andere Sensoren vorzusehen, beispielsweise Lagesensoren oder Kraft- oder Momentensensoren, um die Lage im Raum, Kräfte oder Momente zu bestimmen und zur Steuerung der Ventile einzusetzen.

In der Figur 8a ist eine Schnittansicht der Figur 8 gezeigt. In dem um die Schwenkachse 12 gelenkig an dem Unterteil 13 gelagerten Oberteil 11 und dem Extensionsanschlag 16 ist in der Figur 8a der proximale Ausleger 15 mit der Langlochführung für den proximalen Anlenkpunkt 17 zu erkennen. An dem proximalen Anlenkpunkt 17 ist eine Kolbenstange 420 der proximalen Zylinder-Kolbeneinheit 40 befestigt. An der Kolbenstange 420 ist ein Kolben 410 befestigt, der in einem Gehäuse 400 der Kolben-Zylindereinheit 40 geführt ist. In der dargestellten, vollständig extendierten Stellung des Kniegelenkes 10 befindet sich der Kolben 410 der proximalen Zylinder-Kolbeneinheit 40 an dem proximalen Anschlag. Über nicht dargestellte Hydraulikleitungen, die in den Ventilblock 43 münden und durch die Steuereinheit 44 über entsprechende Ventile geöffnet, vollständig geschlossen oder teilweise geschlossen werden, wird das Hydraulikfluid bei einer Flexionsbewegung von der distalen Zylinderkammer zur distalen Zylinder-Kolbeneinheit 41 geleitet. Ausgehend von der dargestellten Neutralstellung wird bei einer Einbeugung des Kniegelenks 10 Hydraulikfluid in die proximale Zylinderkammer der distalen Zylinder-Kolbeneinheit 41 geleitet, so dass die distale Kolbenstange 420 in Richtung auf die distale Lagerstelle 27 verlagert wird, so dass sich eine Plantarflexion um die Knöchelgelenksachse 22 einstellt.

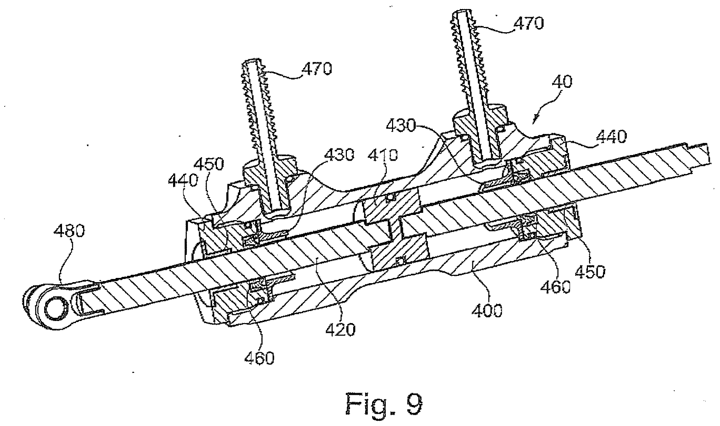

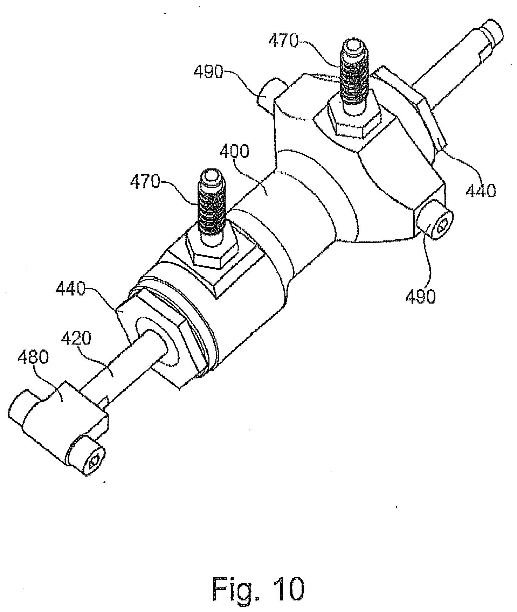

Figur 9 zeigt eine Detailansicht einer Kolben-Zylindereinheit mit einem Gehäuse 400, in dem ein Kolben 410 an einer Kolbenstange 420 befestigt ist. Zwei Endanschlage 430 begrenzen das Volumen, das durch das Gehäuse 400 eingegrenzt werden. Die durch das Gehäuse 400 gebildeten Zylinder sind durch Deckel 440 verschlossen. In den Deckeln 440 sind Gleitlager 450 sowie Abstreifer 460 integriert. An jedem Ende der beiden Zylinder, die durch den Kolben 410 getrennt werden, sind Schlauchanschlüsse 470 vorgesehen, die mit Hydraulikleitungen 42, die nicht dargestellt sind, verbunden werden. An der Kolbenstange 420 ist ein Lagerbolzen 480 angeordnet, der in einem der Anlenkpunkte 17, 27 der Ausleger 15, 25 angeordnet werden kann. Der Lagerbolzen 480 kann als Gelenkkopf mit zwei Achsbolzen für die Lagerung an den Auslegern 15, 25 versehen sein, eine zweite Lagerungsstelle ist in der perspektivischen Darstellung gemäß Figur 10 gezeigt. Die Achsbolzen 490 in dem Gehäuse 400 dienen zur Lagerung in den Seitenplatten entweder des Unterteils 13 oder des Unterschenkelteils 14.

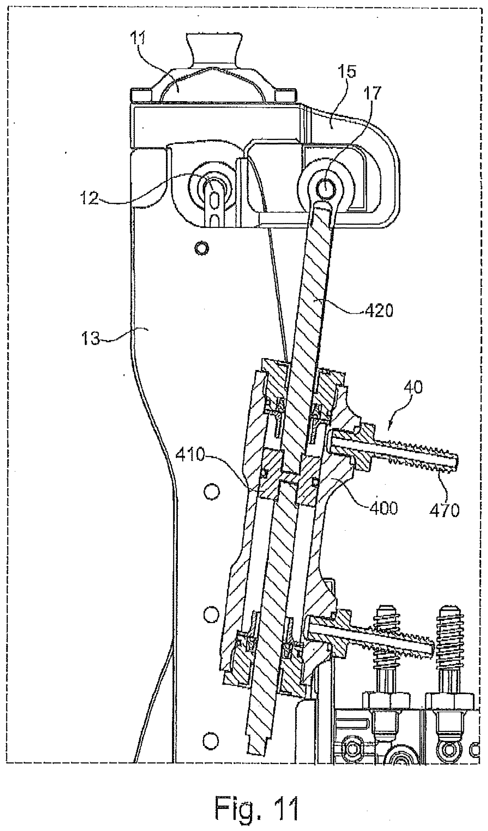

In den Figuren 11 bis 13 ist in einer Schnittdarstellung die proximale Kolben-Zylindereinheit 40 in unterschiedlichen Kniewinkelstellungen gezeigt. In der Stellung, die in der Figur 11 gezeigt ist, beträgt der Kniewinkel 0°, der Kolben 410 innerhalb des Gehäuses 400 befindet sich dann in der Maximalstellung am proximalen Endanschlag. Sämtliches Hydraulikfluid ist aus dem proximalen Zylinder herausgedrückt worden, und das Volumen des distalen Zylinders ist maximal.

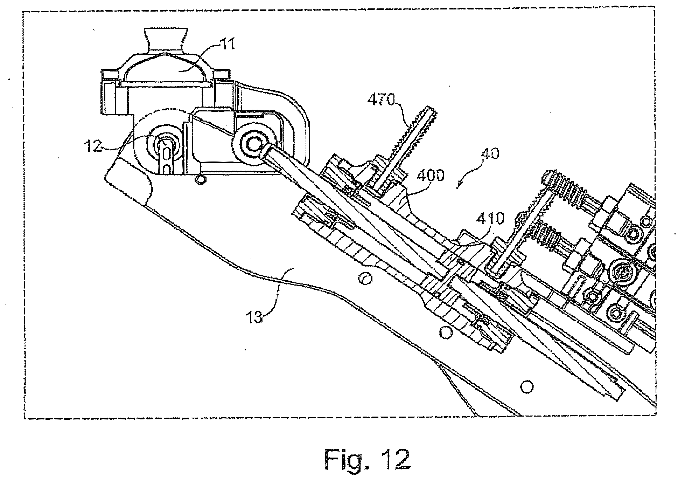

In der Figur 12 ist eine Kniewinkelstellung mit einem Kniewinkel α von 60° dargestellt. Der Kolben 410 befindet sich jenseits der Mitte des Gehäuses 400, so dass der proximale Zylinder ein größeres Volumen als der distale Zylinder aufweist. Durch die Einbeugung des Kniegelenks wurde der Kolben 410 innerhalb des Zylinders nach unten bewegt und Hydraulikfluid aus dem unteren Zylinder durch die Ventile zur nicht dargestellten distalen Kolben-Zylindereinheit 41 geleitet.

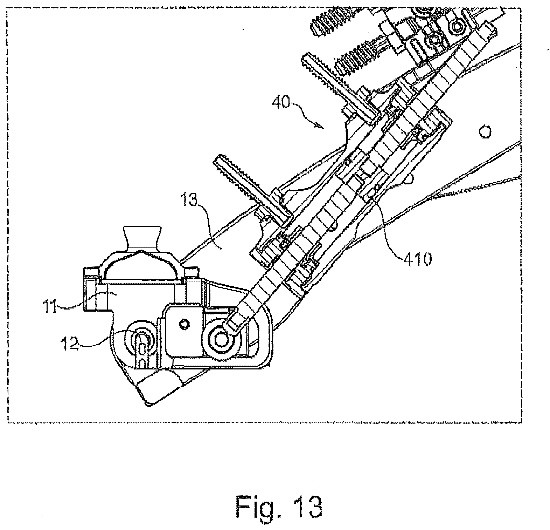

Figur 13 zeigt eine Stellung des Kniegelenks mit einem Kniewinkel α von 120°, wie sie beispielsweise beim Hinknien oder Hocken eingenommen wird. Die Stellung des Kolbens 410 entspricht der der Figur 12, allerdings hat der Kolben 410 bereits einmal den distalen Endanschlag bei einer Stellung von einem Kniewinkel α von 90° erreicht. Die Bewegungsumkehr des Kolbens 410 bei Durchlaufen des Kniewinkels von 90° kann entweder zur Bewegungsumkehr bei der Steuerung des Knöchelgelenks 20 führen oder durch eine Umschaltung der Ventile ausgeglichen werden.

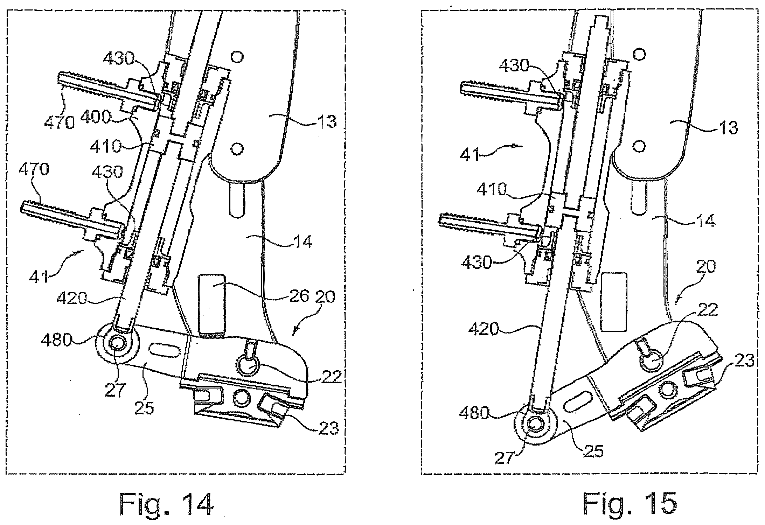

Figur 14 zeigt die Anordnung einer Kolben-Zylindereinheit 41 am Knöchelgelenk 20 mit einer Dorsalflexion, bei der der Kolben 410 nahe dem proximalen Endanschlag 430 positioniert ist. Figur 15 zeigt ein maximal flektiertes Knöchelgelenk 20, beispielsweise bei einem Kniewinkel α von 90°.

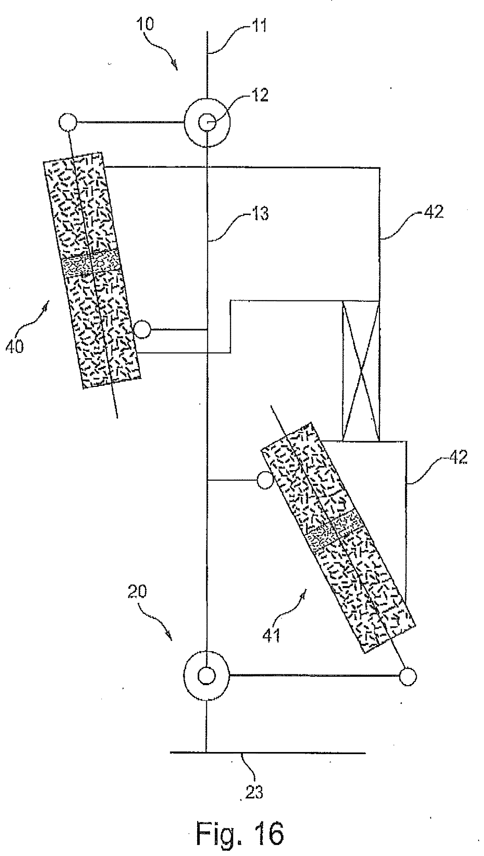

Figur 16 zeigt eine schematische Darstellung unterschiedlicher Verschaltungsmöglichkeiten. Die Schlauchanschlüsse 470 können parallel geschaltet werden, ebenso ist es möglich, dass die Schlauchanschlüsse 470 der distalen Kolben-Zylindereinheit 41 über Kreuz mit den Schlauchanschlüssen 470 der proximalen Kolben-Zylindereinheit 40 verschaltet werden. Ebenso ist es möglich, dass die Kolben-Zylindereinheiten 40, 41 unabhängig voneinander geschaltet werden, so dass keine funktionale Verbindung zwischen den Einheiten vorhanden ist. Das Kniegelenk 10 und das Knöchelgelenk 20 sind somit unabhängig voneinander frei beweglich, gegebenenfalls kann über ein Drosselventil die Dämpfung in dem Kniegelenk 10 und/oder dem Knöchelgelenk 20 eingestellt werden. Wird die proximale Kolben-Zylindereinheit 40 freigeschaltet, während die distale Kolben-Zylindereinheit gesperrt wird, ist das Kniegelenk 10 frei beweglich und das Knöchelgelenk 20 starr, bei einer Sperrung beider Kolben-Zylindereinheiten 40, 41 sind beide Gelenke starr.

Durch die gesteuerte Flexion und Extension im Knöchelgelenk 20 in Abhängigkeit von dem Kniewinkel α und einer Kopplung der Plantarflexion in Abhängigkeit von der Knieflexion ist es möglich, die Bewegungsenergie des Knies für die Knöchelbewegung zu nutzen. Dadurch wird eine Verlängerung der effektiven Beinlänge bei der Kniebeugung am Ende der Standphase erreicht und dadurch die Dauer des Bodenkontaktes verlängert, wodurch die Vertikalbewegung des Körperschwerpunktes während des Ganges minimiert wird. Dies führt zu einer Annäherung an das natürliche Gangbild. Durch die Dorsalflexion in der Schwungphase ist es möglich, die effektive Beinlänge wieder zu verkürzen, um die Stolperwahrscheinlichkeit zu minimieren. Aufgrund der direkten Umsetzung der mechanischen Energie bei minimaler Verlustleistung ist es möglich, eine hohe energetische Effizienz bei einem relativ geringen Gewicht im Vergleich zu bekannten orthetischen Einrichtungen, insbesondere Prothesen mit motorischen Verstelleinrichtungen, zu erzielen.

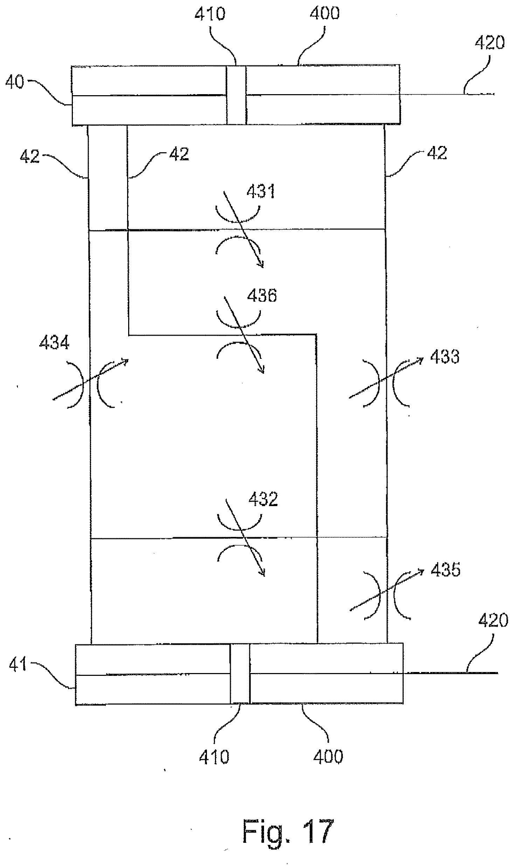

In der Figur 17 sind die beiden Zylinder-Kolbeneinheiten 40, 41, die dem Kniegelenk bzw. dem Knöchelgelenk zugeordnet sind, schematisch in der jeweiligen Neutralstellung dargestellt. Die jeweils aus dem Gehäuse 400 herausragende Kolbenstange 420 ist beispielsweise jeweils an den Auslegern 15, 25 befestigt, um eine Kraftübertragung zu ermöglich, das andere Ende der Kolben-Zylindereinheit ist an dem Unterteil oder Unterschenkelteil gelagert.

Die Hydraulikleitungen 42 verbinden die Zylinderkammern miteinander in Parallelschaltung, die Hydraulikleitungen 42 sind untereinander durch zwei Querleitungen verbunden, eine Diagonalleitung verbindet eine der Kolbenstangen 420 abgewandte Zylinderkammer mit einer der Kolbenstange 420 zugewandten Zylinderkammer der distalen Zylinder-Kolbeneinheit 41.

In jeder der Hydraulikleitungen 42 ist zumindest ein Ventil 431, 432, 433, 434, 435, 436 angeordnet, um unterschiedliche Verschaltungen realisieren zu können. Werden beispielsweise die die Ventile 433, 434, 435 geöffnet und die übrigen Ventile 431, 432, 436 geschlossen, ergibt sich eine Parallelschaltung, die bewirkt, dass eine Verlagerung des oberen Kolbens 410 nach links zu einer Verlagerung des unteren Kolbens 410 nach rechts führt. Um eine gegenläufige Bewegung zu erzeugen, müssen die Zylinder-Kolbeneinheiten 40, 42 kreuzweise verschaltet werden, wozu die Ventile 431, 434 und 435 geschlossen werden, während die Ventile 432, 433 und 436 offen stehen.

Werden die Ventile 433, 434 und 436 geschlossen, führt dies zu einer Entkopplung der proximalen Kolben-Zylindereinheit 40 von der distalen Zylinder-Kolbeneinheit 41.

Durch teilweises Verschließen der geöffneten Ventile 431, 432 und 435 ist es möglich, den Widerstand gegen eine Verlagerung anzupassen.

Wird nur das obere Ventil 431 geöffnet, bleibt das Knöchelgelenk starr, wohingegen das Kniegelenk eingebeugt werden kann, der Widerstand gegen die Einbeugung ergibt sich durch den hydraulischen Widerstand des geöffneten Ventils 431. Ein starres Kniegelenk und ein bewegliches Knöchelgelenk ist möglich, wenn die Ventile 432, 435 geöffnet und die übrigen Ventile geschlossen bleiben.

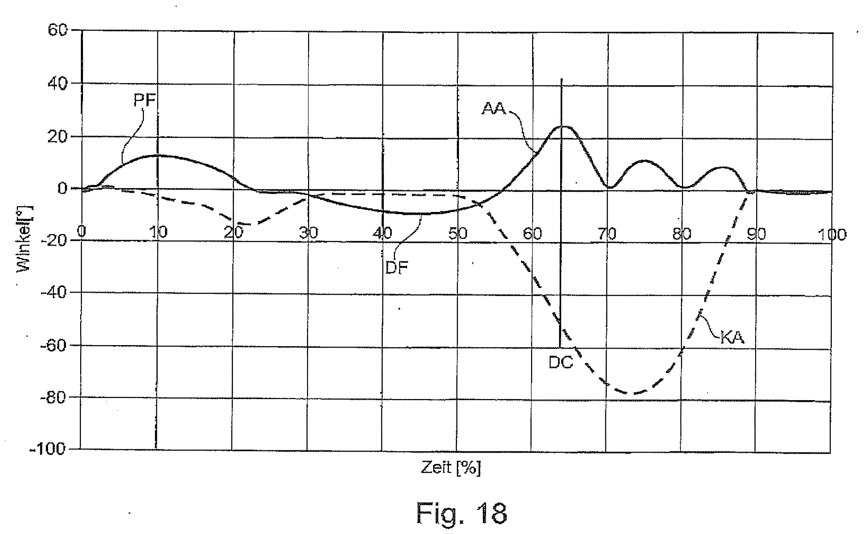

Figur 18 zeigt den Verlauf des Kniewinkels KA und des Knöchelwinkels AA bei einem Prothesenkniegelenk mit einer erfindungsgemäßen Koppeleinrichtung über einen Gangzyklus. Nach dem Aufsetzen der Ferse, dem sogenannten heel strike, ergibt sich eine Plantarflexion PF des Knöchelgelenks entgegen beispielsweise einer Federkraft, die das Knöchelgelenk in der Ausgangsstellung hält. Die Plantarflexion nach dem heel strike wird durch die Übertragung von Kräften von der Knieeinbeugung durch die Kraftübertragungseinrichtung bewirkt und der in Richtung einer Dorsalfelxion wirkenden Kraft der Rückstellfeder überlagert, was im weiteren Verlauf nach ungefähr einem Viertel der Schrittdauer zu einer Aufhebung der in entgegengesetzte Richtungen wirkenden Kräfte führt. Der Knöchelgelenkswinkel ist dann wieder 0°. Beim Kniegelenk ergibt sich eine anfängliche Standphasenflexion bei gleichzeitiger Verringerung des Knöchelgelenkwinkels nach Erreichen eines anfänglichen lokalen Maximums bei ca. 10% der Schrittdauer. Nach Erreichen der maximalen Extension des Kniegelenks wandert nach ungefähr einem Drittel der Schrittdauer der Krafteinleitungspunkt vor das Knöchelgelenk, wodurch es zu einer Dorsalflexion DF kommt. Nach ungefähr der Hälfte der Schrittdauer vergrößert sich der Kniewinkel KA, gleichzeitig wird eine Plantarflexion eingeleitet, die bei der Knieflexion auftretenden Kräfte an das Knöchelgelenk weiterleitet. Nach ungefähr zwei Drittel der Schrittdauer findet eine Entkopplung DC des Knöchelgelenks von dem Kniegelenk statt, so dass das Kniegelenk frei nach hinten schwingen kann, während das Knöchelgelenk nach Erreichen der maximalen Plantarflexion in die Ausgangsstellung zurückbewegt wird. Aufgrund eines nicht vorhandenen Dämpfers erfolgt diese Rückführbewegung in Form einer abklingenden Schwingung.

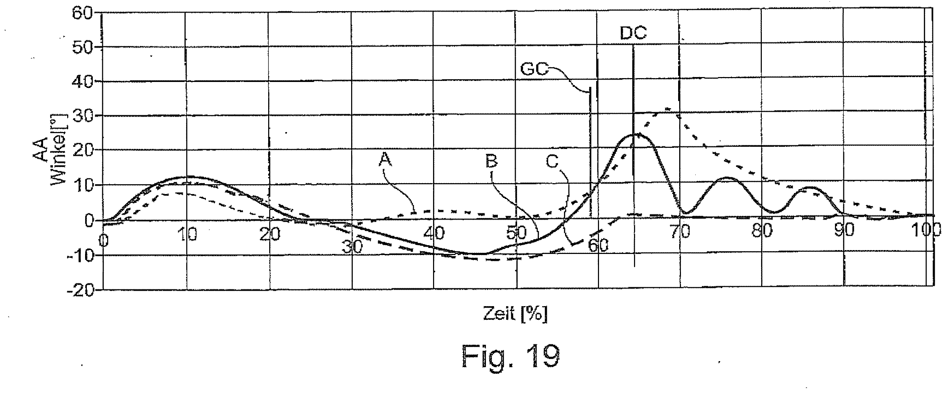

Figur 19 zeigt verschiedene Knöchelgelenkswinkelverläufe über eine Schrittdauer, wobei die Kurven A und B Winkelverläufe zeigen, die mit einer erfindungsgemäßen Vorrichtung erreicht werden, Kurve C zeigt den Verlauf eines herkömmlichen Knöchelgelenks. Bei dem Ende des Bodenkontaktes GC, also am Ende der terminalen Standphase, erreichen die Kurven A und B einen Plantarflexionswinkel zwischen 8° und 10°, während ein herkömmliches Knöchelgelenk eine Dorsalflexion von ca. 8° aufweist. Nach der Entkopplung DC der Kraftübertragungseinrichtung von dem Knöchelgelenk erfolgt eine relativ schnelle Dorsalflexion an der Kurve B, da dort keine Dämpfungseinrichtung vorhanden ist, die Kurve A sieht eine Dämpfung vor, bei der Kurve C wird nach Ende des Bodenkontaktes sehr schnell wieder der Ausgangszustand erreicht.

## Patentansprüche

1. Orthopädietechnische Vorrichtung zur orthetischen oder prothetischen Versorgung eines Patienten mit
• einem Kniegelenk (10), das ein proximales Oberteil (11) und ein schwenkbar daran angeordnetes distales Unterteil (13) aufweist,
• einem Knöchelgelenk (20),
• einem distal an dem Knöchelgelenk (20) befestigbaren verschwenkbaren Fußteil (23), und
• einem zwischen dem Knöchelgelenk (20) und dem Kniegelenk (10) angeordneten Unterschenkelteil (14),
wobei das Oberteil (11) des Kniegelenks (10) oder ein daran befestigtes Oberschenkelteil, das an dem Körper des Patienten festlegbar ist, mit dem Fußteil (23) über eine Kraftübertragungseinrichtung (3; 30, 31, 32, 35; 40, 41, 42) gekoppelt ist, die bei einer Knieflexion eine Plantarflexion des Fußteils (23) bewirkt,
**dadurch gekennzeichnet, dass**
die Kraftübertragungseinrichtung (3; 30, 31, 32, 35; 40, 41, 42) als Zugkraft und/oder Druckkraft übertragende mechanische Koppeleinrichtung in Gestalt eines Gelenkstabes ausgebildet ist.

2. Orthopädietechnische Vorrichtung nach Anspruch 1, **dadurch gekennzeichnet, dass** die mechanische Koppeleinrichtung (30, 31, 32, 35) an einer dorsal oder ventral zu der Knieachse (12) hinausstehenden ersten Lagerungsstelle (15) gelagert und an einer ventral oder dorsal zu der Knöchelgelenksachse zweiten Lagerungsstelle (25) gelagert ist, wobei die Lagerungsstellen (15, 25) auf unterschiedlichen Seiten einer Verbindungslinie zwischen einer Knieachse (12) und einer Knöchelgelenksachse (22) liegen.

3. Orthopädietechnische Vorrichtung nach Anspruch 2, **dadurch gekennzeichnet, dass** zumindest die erste Lagerungsstelle (15) einstellbar ausgebildet ist.

4. Orthopädietechnische Vorrichtung nach einem der voranstehenden Ansprüche, **dadurch gekennzeichnet, dass** die Kraftübertragungseinrichtung (3; 30, 31, 32, 35; 40, 41, 42) eine Kraftübertragung in Dorsalflexionsrichtung sperrt.

5. Orthopädietechnische Vorrichtung nach einem der voranstehenden Ansprüche, **dadurch gekennzeichnet, dass** die maximale Dorsalflexionsstellung des Fußteils (23) variabel einstellbar ausgebildet ist.

6. Orthopädietechnische Vorrichtung nach einem der voranstehenden Ansprüche, **dadurch gekennzeichnet, dass** eine Rückstellvorrichtung dem Fußteil (23) zugeordnet ist, um eine Dorsalflexion des Fußteils (23) zu bewirken.

7. Orthopädietechnische Vorrichtung nach einem der voranstehenden Ansprüche, **dadurch gekennzeichnet, dass** das Knöchelgelenk (20) als elastisches Gelenk ausgebildet ist und eine Rückstellkraft bei einer Auslenkung aus einer Ausgangsstellung ausübt.

8. Orthopädietechnische Vorrichtung nach einem der voranstehenden Ansprüche, **dadurch gekennzeichnet, dass** in dem Knöchelgelenk (20) zumindest ein Dämpfer, der einer Dorsalflexion entgegen wirkt, vorhanden ist.

9. Orthopädietechnische Vorrichtung nach einem der voranstehenden Ansprüche, **dadurch gekennzeichnet, dass** nach Erreichen eines vorbestimmten Beugewinkel eine Dorsalflexion durchgeführt wird.

10. Orthopädietechnische Vorrichtung nach einem der voranstehenden Ansprüche, **dadurch gekennzeichnet, dass** der Gelenkstab dergestalt ausgebildet ist, dass nach Erreichen eines vorbestimmten Kniewinkels keine substanzielle Kraftübertragung oder eine Kraftrichtungsumkehr erfolgt.

## Claims

1. An orthopedic device for the orthotic or prosthetic provision of a patient, with
- a knee joint (10), which has a proximal upper part (11) and a distal lower part (13) arranged pivotably thereon,
- an ankle joint (20),
- a pivotable foot part (23), which can be secured distally on the ankle joint (20), and
- a shin part (14) arranged between the ankle joint (20) and the knee joint (10),
wherein the upper part (11) of the knee joint (10), or a thigh part that is secured thereon and can be fastened to the patient's body, is coupled to the foot part (23) via a force transmission mechanism (3; 30, 31, 32, 35; 40, 41, 42), which causes a plantar flexion of the foot part (23) when the knee is flexed,
**characterized in that** the force transmission mechanism (3; 30, 31, 32, 35; 40, 41, 42) is designed as a mechanical coupling mechanism designed as an articulated rod that transmits tensile force and/or compressive force .

2. The orthopedic device as claimed in claim 1, **characterized in that** the mechanical coupling mechanism (30, 31, 32, 35) is mounted on a first bearing point (15) protruding dorsally or ventrally with respect to the knee axis (12) and is mounted on a second bearing point (25) ventrally or dorsally with respect to the ankle joint axis, wherein the bearing points (15, 25) lie on different sides of a connection line between a knee axis (12) and an ankle joint axis (22).

3. The orthopedic device as claimed in claim 2, **characterized in that** at least the first bearing point (15) is designed to be adjustable.

4. The orthopedic device as claimed in one of the preceding claims, **characterized in that** the force transmission mechanism (3; 30, 31, 32, 35; 40, 41, 42) blocks a force transmission in the dorsiflexion direction.

5. The orthopedic device as claimed in one of the preceding claims, **characterized in that** the maximum dorsiflexion position of the foot part (23) is designed to be variably adjustable.

6. The orthopedic device as claimed in one of the preceding claims, **characterized in that** a restoring device is assigned to the foot part (23) in order to effect a dorsiflexion of the foot part (23).

7. The orthopedic device as claimed in one of the preceding claims, **characterized in that** the ankle joint (20) is designed as an elastic joint and exerts a restoring force in the event of an excursion from a starting position.

8. The orthopedic device as claimed in one of the preceding claims, **characterized in that** at least one damper, which counteracts a dorsiflexion, is present in the ankle joint (20).

9. The orthopedic device as claimed in one of the preceding claims, **characterized in that** a dorsiflexion is performed after a predetermined flexion angle is reached.

10. The orthopedic device as claimed in one of the preceding claims, **characterized in that** the articulated rod is designed that after a predetermined knee angle is reached no substantial transmission of force takes place or a reversal of the direction of force takes place.

## Revendications

1. Dispositif orthopédique pour l'équipement orthétique ou prothétique d'un patient, comportant
• une articulation de genou (10) qui comprend une partie supérieure proximale (11) et une partie inférieure distale (13) agencée mobile en basculement sur celle-ci,
• une articulation de cheville (20),
• une partie de pied (23) mobile en basculement et susceptible d'être fixée à l'articulation de cheville (20), et
• une partie de jambe (14) agencée entre l'articulation de cheville (20) et l'articulation de genou (10),
dans lequel
la partie supérieure (11) de l'articulation de genou (10) ou une partie de cuisse fixée à celle-ci et susceptible d'être immobilisée sur le corps du patient est couplée à la partie de pied (23) via un moyen de transmission de force (3 ; 30, 31, 32, 35 ; 40, 41, 42) qui, lors d'une flexion du genou, provoque une flexion plantaire de la partie de pied (23),
**caractérisé en ce que**
le moyen de transmission de force (3 ; 30, 31, 32, 35 ; 40, 41, 42) est réalisé à titre de moyen de couplage mécanique transmettant une force de traction et/ou une force de pression, sous la forme d'une tige articulée.

2. Dispositif orthopédique selon la revendication 1,
**caractérisé en ce que**
le moyen de couplage mécanique (30, 31, 32, 35) est monté à un premier emplacement de montage (15) dépassant du côté dorsal ou ventral vers l'axe de genou (12) et est monté à un second emplacement de montage (25) ventral ou dorsal par rapport à l'axe de l'articulation de cheville, les emplacements de montage (15, 25) se situant sur différents côtés d'une ligne de liaison entre un axe de genou (12) et un axe d'articulation de cheville (22).

3. Dispositif orthopédique selon la revendication 2,
**caractérisé en ce que**
au moins le premier emplacement de montage (15) est réalisé de façon réglable.

4. Dispositif orthopédique selon l'une des revendications précédentes, **caractérisé en ce que**
le moyen de transmission de force (3 ; 30, 31, 32, 35 ; 40, 41, 42) bloque une transmission de force en direction de flexion dorsale.

5. Dispositif orthopédique selon l'une des revendications précédentes, **caractérisé en ce que**
la position maximale de flexion dorsale de la partie de pied (23) est réalisée de façon réglable variablement.

6. Dispositif orthopédique selon l'une des revendications précédentes, **caractérisé en ce que**
un moyen de rappel est associé à la partie de pied (23), afin de provoquer une flexion dorsale de la partie de pied (23).

7. Dispositif orthopédique selon l'une des revendications précédentes, **caractérisé en ce que**
l'articulation de cheville (20) est réalisée sous forme d'articulation élastique et exerce une force de rappel lors d'une déviation depuis une position de départ.

8. Dispositif orthopédique selon l'une des revendications précédentes, **caractérisé en ce que**
au moins un amortisseur qui s'oppose à une flexion dorsale existe dans l'articulation de cheville (20).

9. Dispositif orthopédique selon l'une des revendications précédentes, **caractérisé en ce que**
après avoir atteint un angle de flexion prédéterminé, il s'effectue une flexion dorsale.

10. Dispositif orthopédique selon l'une des revendications précédentes, **caractérisé en ce que**
la tige articulée est réalisée de telle sorte qu'après avoir atteint un angle de genou prédéterminé, il ne s'effectue sensiblement plus de transmission de force substantielle ou bien il s'effectue une inversion de la direction de la force.
